# EUROPEAN PATENT APPLICATION

(11) **EP 2 258 859 A1**
(43) Date of publication of application: **08.12.2010**
(21) Application number: 09160685.5
(22) Date of filing: 19.05.2009
(51) Int. Cl.: C12N 15/90, A01H 5/00

(54) **Method for performing homologous recombination**

(71) Applicant: Genoplante-Valor, 91025 Evry Cedex (FR)
(72) Inventor: Doutriaux, Marie-Pascale, 91160 Saulx les Chartreux (FR); Dubois, Emeline, 75014 Paris (FR)
(74) Representative: Flesselles, Bruno F.G.

(57) **Abstract**

The invention relates to the generation of plants with improved ability to create recombination events, through inactivation of a dUTPase enzyme.

SEQUENCE LISTING

<110>  GENOPLANTE VALOR

<120>  METHOD FOR PERFORMING HOMOLOGOUS RECOMBINATION

<130>  BRV 10 - EP PRIO

<160>  17

<170>  PatentIn version 3.3

<210>  1
<211>  25
<212>  PRT
<213>  artificial

<220>
<223>  Consensus sequence 1


<220>
<221>  VARIANT
<222>  (20)..(20)
<223>  X is I or V

<220>
<221>  VARIANT
<222>  (20)..(20)
<223>  Xaa is Ile or Val

<400>  1

Ser Ile Asp Val Gly Ala Gly Val Ile Asp Ala Asp Tyr Arg Gly Pro
1               5                   10                  15


Val Gly Val Xaa Leu Phe Asn His Ser
            20                  25


<210>  2
<211>  31
<212>  PRT
<213>  Artificial

<220>
<223>  Consensus sequence 2


<220>
<221>  VARIANT
<222>  (8)..(8)
<223>  Xaa is Leu or Met

<220>
<221>  VARIANT
<222>  (9)..(9)
<223>  Xaa is Val or Ile

<220>

<221>  VARIANT
<222>  (15)..(15)
<223>  Xaa is Val or Ile

<220>
<221>  VARIANT
<222>  (16)..(16)
<223>  Xaa is Ala or Gly

<220>
<221>  VARIANT
<222>  (17)..(17)
<223>  Xaa is Val or Ile

<220>
<221>  VARIANT
<222>  (19)..(19)
<223>  Xaa is His, Gln or Glu

<220>
<221>  VARIANT
<222>  (25)..(25)
<223>  Xaa is Val or Ile

<400>  2

Val Pro Ala Arg Gly Lys Ala Xaa Xaa Pro Thr Asp Leu Ser Xaa Xaa
1               5                   10                  15

Xaa Pro Xaa Gly Thr Tyr Ala Arg Xaa Ala Pro Arg Ser Gly Leu
            20                  25                  30

<210>  3
<211>  137
<212>  PRT
<213>  Artificial

<220>
<223>  Consensus sequence 3

<400>  3

Lys Val Lys Lys Leu Ser Glu Lys Ala Val Ile Pro Thr Arg Gly Ser
1               5                   10                  15

Pro Leu Ser Ala Gly Tyr Asp Leu Ser Ser Ala Val Asp Ser Lys Val
            20                  25                  30

Pro Ala Arg Gly Lys Ala Leu Ile Pro Thr Asp Leu Ser Ile Ala Val
        35                  40                  45

Pro Glu Gly Thr Tyr Ala Arg Ile Ala Pro Arg Ser Gly Leu Ala Trp
    50                  55                  60

Lys His Ser Ile Asp Val Gly Ala Gly Val Ile Asp Ala Asp Tyr Arg

65                  70                  75                  80

Gly Pro Val Gly Val Ile Leu Phe Asn His Ser Asp Ala Asp Phe Glu
                85                  90                  95

Val Lys Phe Gly Asp Arg Ile Ala Gln Leu Ile Ile Glu Lys Ile Val
            100                 105                 110

Thr Pro Asp Val Val Glu Val Asp Asp Leu Asp Glu Thr Val Arg Gly
        115                 120                 125

Asp Gly Gly Phe Gly Ser Thr Gly Val
    130                 135

<210>  4
<211>  166
<212>  PRT
<213>  Arabidopsis thaliana

<400>  4

Met Ala Cys Val Asn Glu Pro Ser Pro Lys Leu Gln Lys Leu Asp Arg
1               5                   10                  15

Asn Gly Ile His Gly Asp Ser Ser Pro Ser Pro Phe Phe Lys Val Lys
            20                  25                  30

Lys Leu Ser Glu Lys Ala Val Ile Pro Thr Arg Gly Ser Pro Leu Ser
        35                  40                  45

Ala Gly Tyr Asp Leu Ser Ser Ala Val Asp Ser Lys Val Pro Ala Arg
    50                  55                  60

Gly Lys Ala Leu Ile Pro Thr Asp Leu Ser Ile Ala Val Pro Glu Gly
65                  70                  75                  80

Thr Tyr Ala Arg Ile Ala Pro Arg Ser Gly Leu Ala Trp Lys His Ser
                85                  90                  95

Ile Asp Val Gly Ala Gly Val Ile Asp Ala Asp Tyr Arg Gly Pro Val
            100                 105                 110

Gly Val Ile Leu Phe Asn His Ser Asp Ala Asp Phe Glu Val Lys Phe
        115                 120                 125

Gly Asp Arg Ile Ala Gln Leu Ile Ile Glu Lys Ile Val Thr Pro Asp
    130                 135                 140

Val Val Glu Val Asp Asp Leu Asp Glu Thr Val Arg Gly Asp Gly Gly
145                 150                 155                 160

Phe Gly Ser Thr Gly Val
                165

<210>  5
<211>  171
<212>  PRT
<213>  Zea mays

<400>  5

Met Ala Ala Ser Asn Gly Ala Ala Ala Val Asp Glu Thr Val Ala Ala
1               5                   10                  15

Thr Asp Ser Val Gln Glu Pro Pro Gln Lys Ile Ser Lys Ile Ser Pro
            20                  25                  30

Leu Leu Lys Val Lys Lys Leu Ser Glu Lys Ala Val Leu Pro Ser Arg
        35                  40                  45

Gly Ser Ala Leu Ala Ala Gly Tyr Asp Leu Ser Ser Ala Glu Glu Met
    50                  55                  60

Val Val Pro Ala Arg Gly Lys Ala Leu Val Pro Thr Asp Leu Ser Val
65                  70                  75                  80

Ala Ile Pro His Gly Thr Tyr Ala Arg Ile Ala Pro Arg Ser Gly Leu
                85                  90                  95

Ala Leu Lys His Ser Ile Asp Val Gly Ala Gly Val Ile Asp Ala Asp
            100                 105                 110

Tyr Arg Gly Pro Val Gly Val Ile Leu Phe Asn His Ser Asp Ala Asp
        115                 120                 125

Phe Ala Val Lys Pro Gly Asp Arg Ile Ala Gln Met Ile Ile Glu Val
    130                 135                 140

Ile Ala Thr Pro Glu Val Ala Glu Val Glu Asp Leu Asp Ala Thr Val
145                 150                 155                 160

Arg Gly Asp Gly Gly Phe Gly Ser Thr Gly Val
                165                 170

<210>  6

<211>  169
<212>  PRT
<213>  Lycopersicon esculentum

<400>  6

Met Ala Glu Asn Gln Ile Asn Ser Pro Glu Ile Thr Glu Pro Ser Pro
1               5                   10                  15

Lys Val Gln Lys Leu Asp His Pro Glu Asn Gly Asn Val Pro Phe Phe
            20                  25                  30

Arg Val Lys Lys Leu Ser Glu Asn Ala Val Leu Pro Ser Arg Ala Ser
        35                  40                  45

Ser Leu Ala Ala Gly Tyr Asp Leu Ser Ser Ala Ala Glu Thr Lys Val
    50                  55                  60

Pro Ala Arg Gly Lys Ala Leu Val Pro Thr Asp Leu Ser Ile Ala Val
65                  70                  75                  80

Pro Gln Gly Thr Tyr Ala Arg Ile Ala Pro Arg Ser Gly Leu Ala Trp
                85                  90                  95

Lys Tyr Ser Ile Asp Val Gly Ala Gly Val Ile Asp Ala Asp Tyr Arg
            100                 105                 110

Gly Pro Val Gly Val Val Leu Phe Asn His Ser Glu Val Asp Phe Glu
        115                 120                 125

Val Lys Val Gly Asp Arg Ile Ala Gln Leu Ile Val Gln Lys Ile Val
    130                 135                 140

Thr Pro Glu Val Glu Gln Val Asp Asp Leu Asp Ser Thr Val Arg Gly
145                 150                 155                 160

Ser Gly Gly Phe Gly Ser Thr Gly Val
                165

<210>  7
<211>  171
<212>  PRT
<213>  Oryza sativa

<400>  7

Met Ala Thr Ala Thr Asn Gly Asn Ala Ser Ala Ala Ala Ala Ala Ala
1               5                   10                  15

Asp Ser Ala Val Gln Glu Pro Pro His Lys Ile Ala Lys Val Ala Pro
            20                  25                  30

Leu Leu Lys Val Lys Lys Leu Ser Glu Asn Ala Val Leu Pro Ser Arg
        35                  40                  45

Gly Ser Ala Leu Ala Ala Gly Tyr Asp Leu Ser Ser Ala Ala Glu Val
    50                  55                  60

Val Val Pro Ala Arg Gly Lys Ala Met Val Pro Thr Asp Leu Ser Ile
65                  70                  75                  80

Ala Ile Pro Glu Gly Thr Tyr Ala Arg Val Ala Pro Arg Ser Gly Leu
                85                  90                  95

Ala Leu Lys His Ser Ile Asp Val Gly Ala Gly Val Ile Asp Ala Asp
            100                 105                 110

Tyr Arg Gly Pro Val Gly Val Ile Leu Phe Asn His Ser Asp Thr Asp
        115                 120                 125

Phe Ala Val Lys Pro Gly Asp Arg Ile Ala Gln Met Ile Ile Glu Val
    130                 135                 140

Ile Val Thr Pro Glu Val Ala Glu Val Glu Asp Leu Asp Ala Thr Val
145                 150                 155                 160

Arg Gly Glu Gly Gly Phe Gly Ser Thr Gly Val
                165                 170

<210>  8
<211>  176
<212>  PRT
<213>  Vitis vinifera

<400>  8

Met Ala Gln His Asp Pro Gln Cys Arg Ser Pro Glu Ile Glu Glu Pro
1               5                   10                  15

Ala Pro Lys Leu Gln Lys Leu His Gln Asn Gly Val Ser Gln Asp Ser
            20                  25                  30

Gln Cys Pro Leu Ser Leu Leu Arg Val Lys Lys Leu Ser Glu Arg Ala
        35                  40                  45

Val Leu Pro Ser Arg Ala Ser Pro Leu Ser Ala Gly Tyr Asp Leu Ser
    50                  55                  60

Ser Ala Thr Glu Thr Lys Val Pro Ala Arg Gly Lys Ala Leu Val Pro
65                  70                  75                  80

Thr Asp Leu Ser Ile Gly Ile Pro Glu Gly Thr Tyr Ala Arg Ile Ala
                85                  90                  95

Pro Arg Ser Gly Leu Ala Trp Lys Tyr Ser Ile Asp Val Gly Ala Gly
            100                 105                 110

Val Ile Asp Ala Asp Tyr Arg Gly Pro Val Gly Val Ile Leu Phe Asn
        115                 120                 125

His Ser Asp Val Asp Phe Glu Val Lys Val Gly Asp Arg Ile Ala Gln
    130                 135                 140

Leu Ile Ile Glu Lys Ile Ile Thr Pro Glu Val Met Glu Val Glu Asp
145                 150                 155                 160

Leu Asp Ser Thr Glu Arg Gly Val Gly Gly Phe Gly Ser Thr Gly Val
                165                 170                 175

<210>  9
<211>  170
<212>  PRT
<213>  poplar trees

<400>  9

Met Pro Pro Ala Asn Leu Gln Asn His Ser Pro Asp Ile Lys Glu Pro
1               5                   10                  15

Ser Pro Lys Val Pro Lys Leu Gln His Asp Asn Ile Pro Ser Tyr Leu
            20                  25                  30

Leu Arg Val Lys Lys Leu Ser Glu Asn Ala Val Leu Pro Ser Arg Gly
        35                  40                  45

Ser Pro Leu Ser Ala Gly Tyr Asp Leu Ser Ser Ala Ser Lys Ala Lys
    50                  55                  60

Val Pro Ala Arg Gly Lys Ala Leu Ile Pro Thr Asp Leu Ser Ile Ala
65                  70                  75                  80

Ile Pro Glu Gly Thr Tyr Ala Arg Ile Ala Pro Arg Ser Gly Leu Thr
                85                  90                  95

Trp Lys His Ser Ile Asp Val Gly Ala Gly Val Ile Asp Ala Asp Tyr
            100                 105                 110

Arg Gly Pro Val Gly Val Ile Leu Phe Asn His Ser Asp Val Asp Phe
        115                 120                 125

Glu Val Lys Val Gly Asp Arg Ile Ala Gln Leu Ile Ile Glu Lys Ile
    130                 135                 140

Val Thr Pro Asn Val Met Glu Val Glu Asp Leu Asp Ala Thr Val Arg
145                 150                 155                 160

Gly Ala Gly Gly Phe Gly Ser Thr Gly Val
                165                 170

<210>  10
<211>  498
<212>  DNA
<213>  Arabidopsis thaliana

<400>  10
atggcttgcg taaacgaacc atcacctaaa ctccaaaagc tcgaccgaaa cggaatccat      60

ggcgattcat caccatcacc attcttcaaa gtgaagaaac tctccgagaa agctgttata     120

ccaacacgcg gctcaccact ctccgctgga tacgatctct caagtgcggt ggattcaaaa     180

gttccagcga gaggtaaagc gcttatccca acggatctaa gtatcgctgt tcccgaagga     240

acttacgcga gaattgcgcc gagatctggt ttggcttgga aacattcgat tgatgttggt     300

gctggtgtga ttgatgctga ttatagagga cctgttggtg tgattttgtt taatcattct     360

gatgctgatt ttgaggttaa gttcggtgat cgaatcgcgc agttgattat cgagaagatt     420

gtgactcctg atgttgttga ggttgatgat ttggatgaga ctgttcgtgg tgatggtggt     480

tttggttcta ctggtgtc                                                   498

<210>  11
<211>  513
<212>  DNA
<213>  Zea mays

<400>  11
atggccgctt ccaacggcgc cgccgcggtc gacgagaccg tcgccgccac cgactccgtg      60

caggagcctc cccagaagat ctccaagatc tctcccctgc tcaaggtgaa gaagctctcc     120

gagaaggcag tgctgccgtc ccgcggctcc gctctcgccg ccggctacga cctctcgagt     180

gccgaggaga tggtggtgcc ggcgcgtggc aaggcgcttg tgccgaccga cctcagcgtc     240

gccatcccgc acggaaccta cgcgcgcatc gcgcccaggt cggggctggc gctgaagcac     300

tccatcgacg tgggcgccgg cgtgatcgac gcggactacc gaggccccgt cggcgtcatc 360

ctcttcaacc actccgacgc cgacttcgcc gtgaagcccg gcgacaggat cgcgcagatg 420

atcatcgagg tgatcgcgac gcccgaggtc gcggaggtgg aggacctcga cgccaccgtc 480

cgtggggacg gagggttcgg gtccaccggc gtc 513

<210> 12
<211> 513
<212> DNA
<213> Oryza sativa

<400> 12
atggccaccg ctaccaacgg caacgcctcc gccgccgccg ccgccgctga ctccgccgtc 60

caggagcccc cgcacaagat cgccaaggtt gctcccttgc tcaaggtcaa gaagctctcc 120

gagaacgccg tcctgccgtc ccgcggctcc gccctcgccg ccggctacga cctgtcgagc 180

gcggcggagg tcgtcgtgcc ggcgaggggg aaggcgatgg tgccgacgga cctcagcatc 240

gccatcccgg agggcaccta cgcgcgcgtc gcgccgaggt ctgggctggc gttgaagcac 300

tcgatcgacg taggcgccgg ggtgatcgac gccgactacc gtggcccggt aggggtcatc 360

ctcttcaacc actccgacac cgacttcgcc gtgaagcccg gcgaccggat cgcgcagatg 420

atcatcgagg tgatcgtgac gccggaggtc gccgaggtgg aggacctcga cgccaccgtc 480

aggggagagg gagggttcgg atccaccggc gtc 513

<210> 13
<211> 31
<212> DNA
<213> Artificial

<220>
<223> Primer

<400> 13
ctagtcgaca tggcttgcgt aaacgaacca t 31

<210> 14
<211> 31
<212> DNA
<213> Artificial

<220>
<223> Primer

<400> 14
ctactgcagt tagacaccag tagaaccaaa a 31

<210> 15
<211> 21
<212> DNA

<213>  Artificial

<220>
<223>  Primer

<400>  15
cttcttcgtc ttacacatca c                                                21


<210>  16
<211>  34
<212>  DNA
<213>  Artificial

<220>
<223>  Primer

<400>  16
gactcgagtc tagatggctt gcgtaaacga acca                                  34


<210>  17
<211>  32
<212>  DNA
<213>  Artificial

<220>
<223>  Primer

<400>  17
gagaattcat cgattcgatc accgaactta ac                                    32

## Description

The invention relates to plant improvement and to the generation of plants with improved properties in their ability to create recombination events, making it possible to either target gene integration or excise unwanted DNA sequences in the genome of said organisms.

Homologous recombination (HR) is useful for specifically inserting sequences at a given target site in a genome. It can also be used to perform allele replacement. This can be used for plant improvement. Furthermore, it is possible to use homologous recombination for nuclear genomic DNA excision, which allows the removal of undesirable exogenous DNA sequences. As an example, in plants, this can be used for removing the selectable marker used in plant transformation, or for the irreversible controlled activation or inactivation of transgenes.
Homologous recombination when integrating a DNA sequence in a genome also solves the problem residing in the random pattern of integration of the heterologous gene into said genome. Indeed, it is known that such random integration may lead to a wide variation in the level of expression of the genes among different transformed organisms after regeneration, thus increasing the cost and lengthening the selection of interesting transformants. Furthermore, it is always possible that these heterologous genes are integrated within endogenous genes, thus disrupting their expression. This is a real problem when these endogenous genes are necessary for the maturation, differentiation and/or viability of the cells or organism.

Gene targeting uses the selection of integration events obtained by homologous recombination between DNA sequences present in the genome of a cell or organism and introduced heterologous DNA sequences. This provides a possibility of controlling the site of integration of these heterologous DNA sequences.
Homologous recombination is nevertheless difficult to achieve in plant cells, which have a greater propensity to mediate non-homologous recombination.
This bad ability to mediate homologous recombination events is also problematic when the transformed organism is difficult to transform, in particular when the *in vitro* culture or regeneration steps of the cells is difficult.
As an example, even though it is now widely possible to transform maize, the rate of transformation is still quite poor and necessitates to perform multiple transformation experiments to obtain an adequate number of transformed plants (transformation rate of about 4%). As the homologous recombination rate in regular maize plants is very low, one can thus conceive that it is very difficult and time consuming to select transformed maize where HR occurred if the system is not improved.

Thus, there is a need for developing systems improving the frequency of homologous events in plants.

As indicated above, homologous recombination can be used for inserting new heterologous sequences, but also for eliminating, removing or substituting sequences (creating new alleles) within the organism genome.

Replacement by homologous recombination of a wild type gene on a chromosome (the target gene) by either a target gene with a new expression cassette, an inactivated gene or a modified gene (new allele) is hampered by the high frequency of random insertion of the whole vector (non-homologous recombination) in animal cells, rather than gene replacement (homologous recombination).
It is possible to use positive/negative selection markers for counter-selection of random insertion events. For example, the thymidine kinase (TK) gene from herpes simplex virus may be used as the negative selection marker. Nevertheless, the vectors are sometimes unstable, and need multiple cloning steps to be made, increasing the time needed to produce them. These vectors are also not so efficient in plants, because the negative marker is often eliminated prior to integration.
It has been reported that creation of a double strand break (DSB) or breaks in the vicinity of the site in which homologous recombination is wished will increase the frequency of HR. These DSB may be created by the use of a restriction enzyme, or a meganuclease, which is a specific restriction enzyme, or zing finger proteins that recognize and cut DNA at long (more than 15 bp) sites.

It is to be noted that homologous recombination can also be used as a tool for removing exogenous DNA sequences that do not contribute to the new phenotype conferred by the transgene, such as selectable marker genes that are necessary for plant transformation and particularly those marker genes that confer herbicide or antibiotic resistance. These DNA sequences to be excised are usually flanked by homologous sequences.
Removal of DNA can also be performed by methods using a restriction enzyme or a mega nuclease to create a double strand break (DSB) or breaks in the vicinity of the duplicated (homologous) DNA, thus allowing efficient DNA excision through homologous recombination. Additionally the creation of DSBs flanking the DNA to be excised allows DNA excision via NHEJ.

There still remains a need for developing new systems for improving the various aspects of homologous recombination in plants.
The invention gives another way to generate DSB, in order to perform homologous recombination.

Indeed, the invention is based on the demonstration that inhibition of the activity of a dUTPase enzyme increases the frequency of homologous recombination events in a plant. It is postulated that this effect is due to the formation of DNA breaks in the genome of the plant.
The invention thus relates, in a first aspect, to a plant presenting inhibition of the expression or activity of a dUTPase enzyme.
According to the invention, a dUTPase (or dUTP pyrophosphatase, EC 3.6.1.23), is an enzyme that catalyzes the hydrolysis of dUTP into dUMP and PPi (pyrophosphate). This enzyme is known to the person skilled in the art and has been demonstrated to be important in preventing the erroneous incorporation of uracil into DNA during replication (Shlomai and Kornberg, 1978). Consistently, Pri-Hadash et al. (1992) have shown that the tomato dUTPase is expressed at a high level (both transcripts and proteins) in the apical vegetative and floral meristematic cells in tomato.
In a preferred embodiment, the sequence of said dUTPase comprises at least one sequence chose among SEQ ID N° 1 and SEQ ID N° 2.
In a more preferred embodiment, the sequence of said dUTPase comprises SEQ ID N° 1 and SEQ ID N° 2.
In another embodiment, the sequence of said dUTPase comprises at least 150 amino acids, and comprises a sequence that is at least 80 %, preferably at least 85 %, more preferably at least 90 %, most preferably at least 95 % identical to SEQ ID N° 3.

Sequence comparisons between two (or more) polynucleotides or polypeptides are typically performed by comparing sequences of two optimally aligned sequences over a segment or "comparison window" to identify and compare local regions of sequence similarity. Optimal alignment of sequences for comparison may be conducted by the local homology algorithm of Smith and Waterman, Ad. App. Math 2: 482 (1981), by the homology alignment algorithm of Neddleman and Wunsch, J. Mol. Biol. 48: 443 (1970), by the search for similarity method of Pearson and Lipman, Proc. Natl. Acad. Sci. (U. S. A.) 85: 2444 (1988), by computerized implementation of these algorithms (GAP, BESTFIT, BLAST N, BLAST P, FASTA, and TFASTA in the Wisconsin Genetics Software Package, Genetics Computer Group (GCG), 575 Science Dr., Madison, WI), or by inspection.
Preferably, the percentage of identity of two polypeptides is obtained by using the Blast program (Blast 2 sequences), using the Blastp program and the BLOSUM62 matrix, with the following parameters: gap open: 11; gap extension: 1; x_dropoff: 0; expect: wordsize: 3; Filter: no. Alignment is performed on the full length of SEQ ID N° 3.

"Percentage of sequence identity" can also be determined by comparing two optimally aligned sequences over a comparison window, where the portion of the polynucleotide or polypeptide sequence in the comparison window may comprise additions or deletions (i. e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the result by 100 to yield the percentage of sequence identity.

In a specific embodiment, said dUTPase is from maize and comprises SEQ ID N° 5, or a sequence that is at least 95 % identical to SEQ ID N° 5.

In a first embodiment, said expression and/or activity of said dUTPase enzyme is inhibited by mutagenesis of the gene coding for said enzyme.
The mutagenesis of the gene encoding the dUTPase can take place at the level of the coding sequence or of the regulatory sequences for expression, in particular of the promoter. It is, for example, possible to delete all or part of said gene and/or to insert an exogenous sequence.
By way of example, mention will be made of insertional mutagenesis: a large number of individuals derived from a plant that is active in terms of the transposition of a transposable element (such as the AC or Mutator elements in corn) are produced, and the plants in which there has been an insertion in the dUTPase gene are selected, for example by PCR.
It is also possible to introduce one or more point mutations with physical agents (for example radiations) or chemical agents, such as EMS or sodium azide treatment of seed, or gamma irradiation. The consequence of these mutations is to shift the reading frame and/or to introduce a stop codon into the sequence and/or to modify the level of transcription and/or of translation of the gene and/or to make the enzyme less active than the wild-type protein. In this context, use may in particular be made of techniques of the "TILLING" type (Targeting Induced Local Lesions IN Genomes; McCALLUM et al., Plant Physiol., 123, 439-442, 2000). Such mutated plants are then screened, in particular by PCR, using primers located in the target gene. One can also use other screening methods, such as Southern Blots or the AIMS method that is described in WO 99/27085 (this method makes it possible to screen for insertion), by using probes that are specific of the target genes, or through methods detecting point mutations or small insertions / deletions by the use of specific endonucleases (such as *Cel* I, *Endo* I, which are described in WO 2006/010646).

In another embodiment, inhibition is obtained by transforming the plant with a vector containing a sense or antisense construct. These two methods (co-suppression and antisense method) are well known in the art to permit inhibition of the target gene.
One can also use the RNA interference (RNAi) method, which is particularly efficient for extinction of genes in plants (Helliwell and Waterhouse, 2003). This method is well known by the person skilled in the art, and comprises transformation of the plant with a construct producing, after transcription, a double-stranded duplex RNA, one of the strand of which being complementary of the mRNA of the target gene.
The invention thus relates to a plant, which is a transgenic plant transformed with a DNA construct comprising a polynucleotide chosen among:
a) a polynucleotide coding for a dUTPase as specified above ;
b) a polynucleotide which has a sequence complementary of the sequence of the polynucleotide of a) ;
c) a fragment of at least 12 consecutive nucleotides of a polynucleotide of a) or b), more preferably at least 20, even more preferably at least 50, the most preferably at least 100 consecutive nucleotides of a polynucleotide of a) or b), or capable of selective hybridization with a polynucleotide of a) or b) ;
d) a polynucleotide containing (i) a polynucleotide X comprising at least 12 nucleotides, preferably at least 15, advantageously at least 20, and even more preferably at least 50, most preferably at least 100 nucleotides capable of selective hybridization with the polynucleotide of a), and (ii) a polynucleotide Y that is complementary of said polynucleotide X, under transcriptional control of an appropriate promoter.
Polynucleotides a) and some polynucleotides c) are suitable for inhibition by co-suppression. Polynucleotides b) and some polynucleotides c) are suitable for inhibition by antisense. Polynucleotides d) are suitable for inhibition by RNAi.

The plant transformed with said DNA construct may contain said polynucleotide within its genome, or be transformed such as expressing it transiently, or through a genetic construct not integrated in the genome. Thus, agro-infiltration or any other way, such as injection or spray, are contemplated for transient expression.

It is foreseen that said nucleic acids are transcribed. They are thus under the control of an appropriate promoter.
Said promoter may be a constitutive, tissue specific, developmentally regulated, inducible or meiosis promoter. Examples of constitutive promoters useful for expression include the 35S promoter or the 19S promoter (Kay et al., 1987), the rice actine promoter (McElroy et al., 1990), the pCRV promoter (Depigny-This et al., 1992), the CsVMV promoter (Verdaguer et al., 1998), the ubiquitin 1 promoter of maize (Christensen et al., 1996), the regulatory sequences of the T-DNA of *Agrobacterium tumefaciens,* including mannopine synthase, nopaline synthase, octopine synthase. Promoters may come from the same species or from another species (heterologous promoters). Although some promoters may have the same pattern of regulation when there are used in different species, it is often preferable to use monocotyledonous promoters in monocotyledons and dicotyledonous promoters in dicotyledonous plants. "Meiosis promoter" means a promoter which may allow the transcription of the operably linked sequence within cells undergoing "meiosis". Examples of meiosis promoter are the promoter Lim10 from *Lilium longifolium* (Morita et al., 2003), AtXrcc3 (Bleuyard and White 2004), Λ/CDC45 (Stevens et al., 2004), /AtDMC1 from *Arabidopsis thaliana* or OsDMCI from *Oriza sativa,* (Klimyuk and Jones, 1997 ; Doutriaux et al., 1998 ; Siaud et al, 2004 ;), AtMEM (US 6,476,297). Such a meiosis promoter should be ideally meiosis-specific or preferentially active during meiosis.
Indeed, the inventors postulate that the efficiency of homologous recombination may be better when the cell is at the meiosis stage. "Meiosis" is the specific cell division leading to gametes production, and the inventors believe that meiosis is a preferred phase for recombination in plants, even though recombination may also occur during mitosis.

According to another embodiment, expression of said nucleic acid inhibiting expression of the dUTpase may be inducible. By "inducible", it is meant that the transcription of the polynucleotide only becomes active in response to an external stimulus. This stimulus may be a chemical or mechanical stimulus. In this preferred embodiment, said nucleic acid inhibiting expression of the dUTpase is under the control of an inducible promoter. As an illustration, the inducible promoter may be induced by a stress or a chemical agent.
Inducible promoters may be induced by pathogens or wounding, more preferably they are induced by abiotical stress like cold, heat, UV light, high salt and water deficit. Promoters useful for targeted expression in transgenesis are reviewed in Potenza et al., 2004. Some abiotic stress promoters are the *Arabidospsis thaliana* or *Oriza sativa* DREB genes promoters (Dubouzet et al., 2003 ; Lee et al., 2004 ; Pellegrineschi et al., 2004) ; the *Oriza sativa* SISAP1, CDPK7 or WSI gene promoters (Mukhopadhyay et ai, 2004 ; Saijo et ai, 2000; Takahashi et al., 1994) the *A. thaliana* rd29 gene promoters (Yamaguchi-Shinozaki and Shinozaki 1993). Some plant heat inducible promoters may also be used /?sp18.2 or hspλ Oλ from *A. thaliana* (Yoshida et al., 1995 ; Young et al., 2005 ), /?sp17.6 or hspM.2, from Glycine max (Severin and Schoffl, 1990 ; Saidi et al., 2005). DNA microarrays have been used to identify stress regulated sequences (Rabbani et ai, 2003 ; EP 1 452 596; WO 02/16655) The signalisation pathway of the response to stress includes abscisic acid signalisation so ABA-inducible promoters may also be powerful stress-inducible promoters, such as the Horgum vulgare A22 and hval promoters (Shen et al., 1993 ; Straub et ai., 1994), Zea *maize* rab 17, DBF1 and DBF2 (Villardel et ai., 1990 ; Kizis and Pages, 2002), *Arabidopsis thaliana* ABF3 (Genbank accession AK175851 ), and *Oriza sativa* rab21 (Mundy and Chua, 1988).

In another embodiment, the foreseen promoters are induced by chemicals (for review, see Moore et al., 2006, Padidam M. 2003 and Wang et al., 2003 and Zuo and Chua 2000). Some examples of couples of chemically inducible systems and chemical inducer used in plants are, the alcA promoter from *A. nidulans,* inducible by the Ethanol (Roslan et al., 2001 ; Deveaux et al, 2003) or the ecdysone receptor from C. *fumiferana,* inducible by an ecdysone agonist (Koo et al., 2004).

In another embodiment, expression of said nucleic acid inhibiting expression of the dUTPase is indirectly induced by a chemical. As an illustration, one can use the GVG gene, coupled with the UAS promoter. The GVG gene codes for a modified rat glucocorticoid responsive transcription factor that remains in the plant cytosol as a complex. On dexamethasone application, this complex dissociates such that the GVG protein enters the nucleus and binds to the target DNA sequences (UAS). Transcription from the UAS promoter allows the production of the polynucleotide inhibiting the dUTPase expression. This is considered as a dexamethasone inducible (although indirectly) promoter used to control transcription of said nucleic acid inhibiting expression of the dUTpase (Aoyama and Chua (1997)).

The invention also relates to a method for improving homologous or homeologous recombination in a target plant as compared to a control plant, comprising totally or partially inhibiting expression and/or activity, in said target plant, of a dUTPase, wherein said expression and/or activity is not inhibited in said control plant.

Inhibition of the dUTPase in the target plant in the above methods is performed, in particular, by using of at least one polynucleotide chosen among:
a) a polynucleotide coding for a dUTPase as defined above ;
b) a polynucleotide which has a sequence complementary of the sequence of the polynucleotide of a) ;
c) c) a fragment of at least 12 consecutive nucleotides of a polynucleotide of a) or b), more preferably at least 20, even more preferably at least 50, the most preferably at least 100 consecutive nucleotides of a polynucleotide of a) or b), or capable of selective hybridization with a polynucleotide of a) or b) ;
d) a polynucleotide containing a polynucleotide X comprising at least 12 nucleotides, preferably at least 15, advantageously at least 20, and even more preferably at least 50, most preferably at least 100 nucleotides capable of selective hybridization with the polynucleotide of a), and a polynucleotide Y that is complementary of said polynucleotide X.
The invention also relates to an expression cassette comprising a polynuleotide as defined above, under transcriptional control of an appropriate promoter. Said promoter is a constitutive, tissue specific, developmentally regulated, inducible or meiosis promoter, as described above.
A vector comprising the expression cassette according to the invention is also part of the invention.
Said vector is in particular used to perform transformation of the target plant with the cassette of the invention. Said vector is thus preferably adapted to *Agrobacterium* mediated transformation. Such vectors (and transformation method) are well known in the art. These vectors contain in particular a left border and a right border, the DNA between which integrating into the genome of the target plant. They also contain appropriate origins of replication and marker genes to be multiplied in bacteria.

In a specific embodiment, said vector further comprises a selectable marker gene under the control of an appropriate promoter.
In another embodiment, said expression cassette containing the nucleic acid inhibiting expression of the dUTPase and said selectable marker gene are flanked by homologous sequences Y5' and Y3'.
Two sequences are said to be homologous if they present more than about 80%, preferably more than 90% identity, whereas identity between nucleic acid sequences is calculated by the methods described above. Preferably, sequences Y5' and Y3' are identical.
Preferably, sequences Y'3' and Y'5' comprise at least 50, preferably 100 nucleotides, more preferably at least 200 nucleotides, more preferably at least 500 nucleotides, most preferably at least 1000 nucleotides or 2000 nucleotides.

Such vector can be used in a method for excising the selectable marker gene. In this case, the vector may also contain a transgene (gene of interest under the control of an appropriate promoter), that is located outside the region between the Y3' and Y5' sequences.
The method for excising a sequence from the genome of a plant is described in details below.

The invention thus makes it possible to excise a DNA fragment Z in the genome of a plant, wherein said DNA fragment Z is flanked by homologous sequences Y3' and Y5' respectively at its 3' and 5' ends, comprising the steps of providing to said cells a polynucleotide according to the invention, thereby leading to excision of said DNA fragment Z from the genome of said plant by recombination between said sequences Y3' and Y5'.
As mentioned above, it is preferred that sequences Y3' and Y5' are, at least 80 % preferably at least 90 %, more preferably at least 95 %, more preferably at least 98 %, even more preferably at least 99 % identical, or are identical.
It is also preferred that these sequences are at least 50 nucleotides long, more preferably at least 100 nucleotides long, even more preferably at least 200 nucleotides long, even more preferably at least 500 nucleotides long, most preferably at least 1000 nucleotides long, even more preferably 2000 nucleotides long.
Use of an inducible or meiosis specific promoter is preferred.

In this embodiment, the purpose is to eliminate the specific DNA fragment by performing a double-strand break, reattachment / repair of this DSB leading to excision of this specific DNA fragment.
In a preferred way of realization of the invention, the attachment of said DSB is performed by homologous recombination (HR) between said homologous Y and Y' sequences, and leads to the excision of the DNA fragment and one copy of the recombinated Y3' or Y5' sequence.

It is also to be noted that excision of sequence Z can also occur when said attachment of said cut ends (repair of DSB) is performed by non homologous end joining (NHEJ) and at least part of the sequences flanking said DSB- generated fragments are attached together. Non homologous end joining is also known as "illegitimate recombination": this method results from the attachment of the two DNA strands produced by the DSB "double strand break"; no homologous region are necessary for this method but homologous regions may be present on each DNA strand.

As indicated above, in specific embodiments of the present invention, said DNA fragment Z to be eliminated comprises a chimeric gene consisting of a promoter linked to a selectable marker gene (or phenotypic marker gene) and a polyadenylation sequence.
In a preferred embodiment, the selectable marker gene gives resistance to an antibiotic or an herbicide to said plant. More preferably the selectable marker gene comprises of a gene chosen in the group consisting of the pat, the bar or the nptll genes. In the present invention, the term "selectable marker", "selectable gene", "selectable marker gene", "selection marker gene", "marker gene" are used interchangeably. These selectable markers include, but are not limited to, antibiotic resistance genes, herbicide resistance genes or visible marker genes.
Other phenotypic markers are known in the art and may be used in this invention. A number of selective agents and resistance genes are known in the art. (See, for example, Hauptmann et al., 1988 ; Eichholtz et al., 1987 ; and Meijer et al., 1991 ). Notably the selectable marker used can be the bar or pat genes conferring resistance to bialaphos (White et al., 1990), the sulfonamide herbicide Asulam resistance gene, sul (described in WO 98/49316) encoding a type I dihydropterate synthase (DHPS), the nptll gene conferring resistance to a group of antibiotics including kanamycin, G418, paromomycin and neomycin (Bevan et al., 1992), the hph gene conferring resistance to hygromycin (Gritz et al., 1983), the EPSPS gene conferring tolerance to glyphosate (US 5,188,642), the HPPD gene conferring resistance to isoxazoles (WO 96/38567), the gene encoding for the GUS enzyme, the green fluorescent protein (GFP), expression of which, confers a recognisible physical characteristic to transformed cells, the chloramphenicol transferase gene, expression of which, detoxifies chloramphenicol. Advantageously, the selectable marker gene is inserted between a promoter and a terminator.

According to this advantageous embodiment, the marker gene is preferably controlled by a promoter which allows expression in cells, thus allowing selection of cells or tissue containing the marker at any stage of development of the plant. Preferred promoters are the promoter of nopaline synthase gene of Agrobacterium, the promoter derived from the gene which encodes the 35S subunit of cauliflower mosaic virus (CaMV) coat protein, and the rice actin promoter. However, any other suitable second promoter may be used. Any terminator may be used. Other elements like introns and enhancers can also be present in the nucleic sequence of interest in order to improve the expression of the gene of interest. One could in particular note the FAD2 intron from Arabidopsis described in WO 2006/003186.
In this specific embodiment, one can obtain a transformed plant by the use of the marker gene selection and, in a further step remove the marker gene by the protocol of the invention, the marker gene being located on the fragment Z. In this particular protocol the polynucleotide inhibiting the dUTPase activity may be controlled, for example through use of a meiotic promoter, or of a dexamethasone inducible promoter.

If the excised fragment contains a sequence encoding a selectable marker the absence of this marker may be the way for identification of these plants. "Negative markers" may also be used for this selection. A "negative marker" is a marker the presence of which is deleterious to the plant generally in the presence of an inducer, so that the selection of the plant on said inducer (selective agent) allows the selection of plants in which the excision has occurred and said negative marker has been deleted. Example of "negative markers" are reviewed in Miki and McHugh (2004), as cytosine deaminase (Babwah et al., 2000 ; lida and Terada 2005), or diphtheria toxin A (Terada et al., 2004). Another negative marker is D-amino acid oxidase (Erikson et al., 2004). These negative markers may be under the control of an inducible promoter to allow the development of plants before the excision step. In this particular way of achieving the invention, one can transform a plant with the given construct containing the sequence encoding for the negative marker under the control of an inducible promoter. The plant may be retransformed or crossed with a plant containing a sequence encoding the polynucleotide inhibiting expression of the dUTPase. The transcription of this polynucleotide allows the excision of the DNA fragment according to the invention. Selection on a specific selective agent selects against plants that still contain the negative selection marker and allows the selection of the plants where the excision has occurred.

As mentioned above, in a preferred embodiment of the invention, the sequences encoding the polynucleotide leading to inhibition of the dUTPase activity are located within said fragment to be excised.
In this embodiment, it may also be useful for the sequence encoding the polynucleotide leading to inhibition the dUTPase activity to be under the control of an inducible or a tissue or developmentally regulated promoter.

It is also possible to eliminate the sequences inactivating dUTPase activity by crossing the plants and segregating the ones that have lost the construct, in particular by PCR.

It is to be noted that, in another embodiment, excision of the DNA fragment, in particular by homologous recombination can lead to production of an expressible polynucleotide. The method of the invention thus makes it possible to have conditional expression of a given gene, especially when the the polynucleotide leading to inhibition the dUTPase activity is under the control of an inducible promoter and is located within the DNA fragment to be excised. In this embodiment, the expression cassette used in this invention, may contain from 5' to 3': a promoter, a first polynucleotide Y1, a polynucleotide Y, said sequence Z to be excised, another polynucleotide Y, a second polynucleotide Y2, and a terminator sequence. In this embodiment, the polynucleotide Y1YY2 formed after removal of sequence Z by homologous recombination between the two Y sequences makes a complete coding sequence imparting a desired phenotype in said plant.

For irreversible control activation of a protein, the fragment to be excised is inserted in the sequence encoding for a protein in such a way, that the interrupted sequence will not lead to a correctly transcribed RNA and a functional protein. The insertion may be done in the coding sequence but also in an intron or in the promoter. In this embodiment, the excision of the target fragment allows the correct expression of the protein.

The plant containing the DNA fragment to be excised may be retransformed or crossed with a transgenic line containing the sequence encoding the polynucleotide leading to inhibition of the dUTPase. Excision of the fragment may be observed in the lineage of the plant containing both constructs. When the polynucleotide leading to inhibition of the dUTPase is under the control of an inducible promoter, induction of its expression in the plant containing both constructs allows the precise production of the given protein.
For this embodiment, one useful stimuli may be high or cold temperature. In such a mode of realization the invention offers a new binary system for control expression of protein. Such system may be of interest for example for the production of recombinant proteins of therapeutics usage.
For this particular usage, the polynucleotide leading to inhibition of the dUTPase may also be under the control of a tissue specific promoter to induce the expression of a recombinant protein in this related tissue. Examples of tissue specific promoters may be root specific, such as the rcc2 or rcc3 promoters (Xu et al., 95), seed specific such as the HMWG (Norre et al., 2002) and Zein promoters (Russell and Fromm 97) or leaf specific such as the rubisco (Bansal et al., 92) or PEPC promoters (Matsuoka et al, 1994).

Plant with an excised DNA fragment can be identified by any mean known by a person skilled in the art. Such means include PCR, Southern, Northern or Western hybridizations designed to detect the loss of the excised fragment.

Plants in which an excision by HR according to the invention occurs may also be selected by the use of traditionally used selection markers. For this specific embodiment, the construct used may contain in the following order (from 5' to 3') a promoter, a first polynucleotide Y1 , a polynucleotide Y, the sequence X to be excised, a second polynucleotide Y, a polynucleotide Y2 and a terminator sequence. In this example the polynucleotide Y1YY2 obtained after excision of the DNA fragment encodes for a selection marker. The use of this selection marker makes it possible to select the plants where the fragment excision occurs. Examples of such markers have been described above, such as gene coding for resistance to herbicide.

In another embodiment, the invention relates to methods of gene targeting and insertion of genes within the genome of an organism, in particular a plant, using the improvement of homologous recombination through the induction of double strand breaks by inhibition of a dUTPase, according to the invention.

The invention thus relates to a method for integrating a DNA sequence X at a predetermined location within the genome of a plant, wherein said location is flanked by a sequence Y3' and a sequence Y5' respectively at its 3' and 5' ends comprising the steps of
a) providing to cells of said plant a nucleic acid comprising said DNA sequence X flanked by sequences Y'3' and Y'5', respectively homologous to said sequences Y3' and Y5', located respectively at its 3' and 5' ends,
b) providing to said cells a polynucleotide as defined above thereby leading to integration of said DNA sequence X within the plant genome by homologous recombination between said Y3' and Y5' sequences and said Y'3' and Y'5' sequences.
Preferably, sequences Y'3' and Y'5' comprise at least 50, preferably 100 nucleotides, more preferably at least 200 nucleotides, more preferably at least 500 nucleotides, most preferably at least 1000 nucleotides or 2000 nucleotides.
Preferably, sequences Y'3' and Y'5' are identical to sequences Y3' and Y5' that are in the genome of the plant.

Providing and expressing the polynucleotide according to the invention will inhibit expression of the dUTPase in the cells of the plant, thereby leading to formation of double-strand breaks, which will improve the homologous recombination between the genomic and the external sequences.
In a preferred embodiment, said polynucleotide in step b) is provided via integration, within cells of said plant, of the expression cassette of the invention. Specifically, integration can be performed by transformation of the receiving host with a vector according to the invention.
Transient expression systems are also foreseen, as described above.
In another embodiment, said polynucleotide in step b) is provided by crossing a transgenic plant containing said nucleic acid comprising said DNA sequence X flanked by sequences Y'3' and Y'5' respectively at its 3' and 5' ends, with another transgenic plant containing the sequence of said polynucleotide in step b).
In these methods, it is preferred to use an inducible or meiosis specific promoter to drive transcription of the nucleotide inhibiting expression / activity of the dUTPase.

As described above, when applying these methods to a whole plant, it is preferred that transcription of the polynucleotide inhibiting the dUTPase is made active during meiosis. This may be obtained by use of meiosis-specific or inducible promoters and application of the inducers during meiosis.

As previously indicated, the described methods may be used in any plant. It is particularly useful on dicotyledonous and monocotyledonous plants, preferably for monocotyledonous, including but not limited to maize, wheat, barley, rice, rye, oat, trticale. Tomato, pepper, lettuce, beet, carrots, melon, rape, sunflower are also plants (list non limitative) in which the method of the invention may be performed. The invention also encompasses transformed plants containing the elements described above, or being obtained by methods of the invention. The invention also encompasses cells of plants containing the elements described above, or being obtained by methods of the invention.
In a preferred embodiment, said plant is a monocotyledonous plant. In another embodiment, said plant is a dicotyledonous plant, and in particular from the *Brassica* species, specifically *Brassica napus.*
In a preferred embodiment, said plant is a cereal. In particular, said plant is maize. In another embodiment, said plant is rice. In yet another embodiment, said plant is wheat.
In another embodiment, said plant is cotton. In yet another embodiment, said plant is soybean.

Transgenic lines containing the polynucleotide of the invention are obtained by traditional methods for genetic transformation of plants and can be obtained by any one of the techniques known to one skilled in the art: methods of direct transfer of genes such as direct micro-injection into plant embryoids, vacuum infiltration (Bechtold et al. 1993) or electroporation (Chupeau et al., 1989) or the bombardment by gun of particules covered with the plasmidic DNA of interest (Finer et al., 1992). Agrobacterium mediated transformation methods may also be used *Agrobacterium tumefaciens,* in particular according to the method described in the article by An et al., (1986), or in particular according to the method described in the article by Guerche et al., (1987). According to a preferred mode, it is possible to use the method described by Ishida et al., (1996) for the transformation of maize.

The present invention will be further understood in view of the annexed figures and following examples.

### FIGURES

Figure 1: AtDut1 complements the *E*. *coli dut1* mutant (A) and the *S*. *cerevisiae dut1* mutant (B) for their sensitivity to 5-fluoro-uracil (50µM).
Figure 2: Scheme of the RNAi constructs. RNAi constructs were established in pKannibal, then a Spel-Sacl fragment (containing p35S + the RNAi construct) was cloned into the same sites of the T-DNA vector pPF111.
Figure 3: The RNAi/DUT1 plants are sensitive to 5FU (50µM). Progeny from 2 independent RNAi/DUT1 (#4, #5) were grown on MS and MS + 5FU (50µM).
RNAi/0 is not affected on MS + 5FU
Figure 4: A measure of dUMP production following dUTP addition to protein extracted from RNAi/0 and RNAi/*DUT1* plants
Figure 5: The Luciferase assay, as in Molinier et al, Plant Cell (2004): schematic representation of the recombination Luciferase substrate.
Figure 6: A measure of somatic homologous recombination in plants (T3) transformed by the RNAi/0 (517) or RNAi/DUT1 (562) construct. 7 independent lines were used. Somatic recombination is assessed as the % of plants presenting 0, 1, 2, 3, 4, or 5 luciferase foci.

### EXAMPLES

### Example 1: Characterization of a dUTPase gene in Arabidopsis Bacterial strains and media

*E. coli* strains AB1157 (wt) and AK105 (AB1157 *dut1*) were previously described by Kouzminova and Kuzminov (2004). For the *dut1* complementation assay, At*DUT1* (SEQ ID N° 10) was cloned into pSE380 (Promega) at the *Sa*/I (Nter) and Pstl (Cter) sites following PCR amplification with primers ctagtcgacATGGCTTGCGTAAACGAACCAT (SEQ ID N° 13) and ctactgcagTTAGACACCAGTAGAACCAAAA (SEQ ID N° 14). 5-Fluoro-uracil was added to LB medium at 50µM final concentration.

*S*. *cerevisiae* strains FF18733 (wild type) and BG217 (*dut1*) were previously described by Guillet et al 2006. For the *dut1* complementation assay, AtDUT1 was cloned into p415GAL1 (Guillet and Boiteux, 2003). 5-Fluoro-uracil was added to yeast medium at 50µM final concentration.

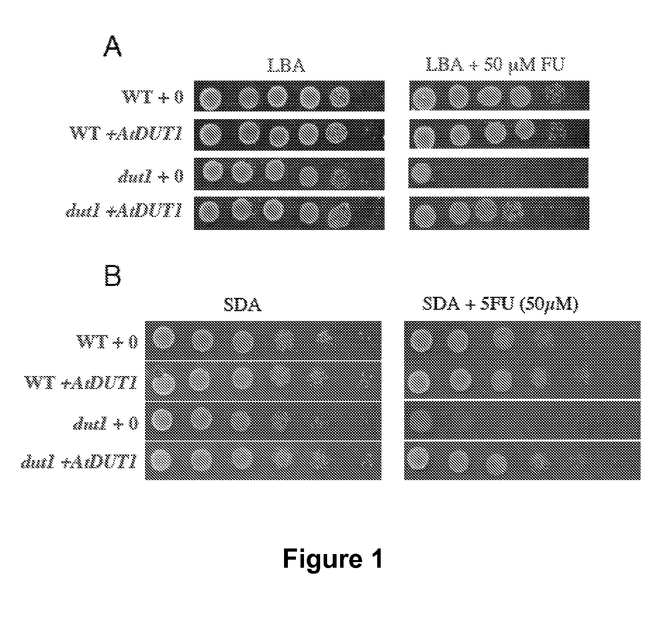

Constructs carrying the At*DUT1* gene in a plasmid providing expression either in *E*. *coli* (pSE380) or in yeast ((p415GAL1) were introduced into the respective wt or *dut1* mutant strains. Complementation of the *dut1* character was assessed following growth of the transformed strains onto 5FU-containing media, as *dut1* mutants are sensitive to this base analog. It can be concluded the Figure 1 that complementation of both *E*. *coli* or *S*. *cerevisiae* mutants was effective.

Figure 1 demonstrates that introduction of the At*DUT1* gene into *E*. *coli* or *S. cerevisiae dut1* mutants restored wild type growth of the *E*. *coli dut1* mutant on uracil or 5FU containing media.

### Example 2: construction of RNAi constructs to inactivate AtDUT1 expression in planta

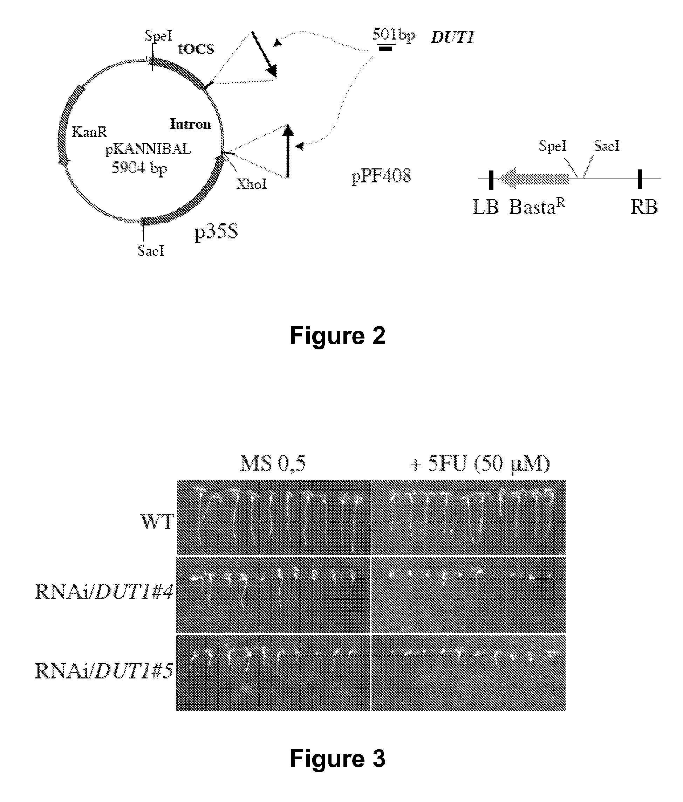

RNAi constructs were made in pKannibal as previously described (Siaud et al., 2004) and subsequently transferred into T-DNA vectors (Figure 2). To be under the control of the p35S promoter the RNAi construct, comprising both the p35S promoter and the Nos terminator, was subcloned into pPF111 (Sacl-Spel restriction/ligation) a pPZP derivative in which the Basta resistance cassette has been cloned. This plasmid provides a BastaR cassette (Siaud et al 2004). The RNAi construct-containing T-DNA vectors were electroporated into Agrobacterium strain GV3101 (Koncz and Schell, 1986). Transformation of Arabidopsis, ecotype WS, was carried out as previously described (Clough et al., 1998). Transformed T1 plants were selected using Basta under greenhouse conditions. Presence of the RNAi construct in the Basta-resistant plants was checked by PCR amplification of their genomic DNA using a primer specific for the pKannibal intron region (Oligo41 CTTCTTCGTCTTACACATCAC, SEQ ID N° 15) and another primer specific for the relevant construct.
Primers used for cloning into pKannibal vectors are: K68-Up-Xhol-Xbal GACTCGAGTCTAGATGGCTTGCGTAAACGAACCA (SEQ ID N° 16) and K68-Do-EcoRl-Clal GAGAATTCATCGATTCGATCACCGAACTTAAC (SEQ ID N° 17) (K68 clone).
To check for their 5FU sensitivity, transgenic plants carrying the RNAi/*DUT1* cassette were grown *in vitro* by sewing seeds on MS 0.5 (Duchefa, NL). p35S::RNAi/At*DUT1* transformed plants were grown on MS 0.5 supplemented with 5FU (50µM). Figure 3 shows that RNAi/At*DUT1* plants are sensitive to 5FU, as their growth is considerably reduced on this medium compared to regular MS that does not contain the 5FU base analog.

### Example 3: RNAi/DUT1 plants are inhibited in the dUTPase activity

Total protein are extracted from 0,3 g of leaves from two months old plants. Leaves are collected and crushed in the presence liquid nitrogen and then 1 ml of extraction buffer is added (100 mM Tris-HCl (pH 7,5), 20 mM MgCl2, 5 mM DTT and 1 µl of protease inhibitor cocktail (Sigma-Aldrich, L'ISLE D'ABEAU CHESNE, France)). The suspension is centrifuged at 14 000 g for 10 minutes at 4°C and supernatants are dessalted on a NAP-5 column (GE-Healthcare) and eluted with extraction buffer.
Protein concentrations in the extracts are measured according to Bradford and collaborators (1976). The dUTPase activity assay is performed in 100 mM Tris-HCl (pH 7,5), 20 mM MgCl2, 100 µM dUTP (Sigma-Aldrich). The reaction is initiated by adding protein extracts and then set at 30°C for increasing lengths of time. The reaction is stopped by heating at 100°C. After centrifugation, the supernatant is subjected to reverse-phase ultra performance chromatography (AcquityTM UPLC, Waters) using a UPLC HSS T3 1.8 µm (100 mm•2.1 mm i.d.) column (Waters). dUMP formed by the reaction is eluted using a linear gradient of 5% methanol in 15 mM Triethylamine pH7.9 (0.6 mL.min-1) and detected at 260 nm using a diode array detector (Waters 2696 PDA detector). Peak areas are calculated using the Empower software (Waters), using authentic dUMP marker as standard (Sigma).
To ensure that steady-state conditions were applied, at least three time points were used for determination of product. Product formation was linear for at least 45 min.

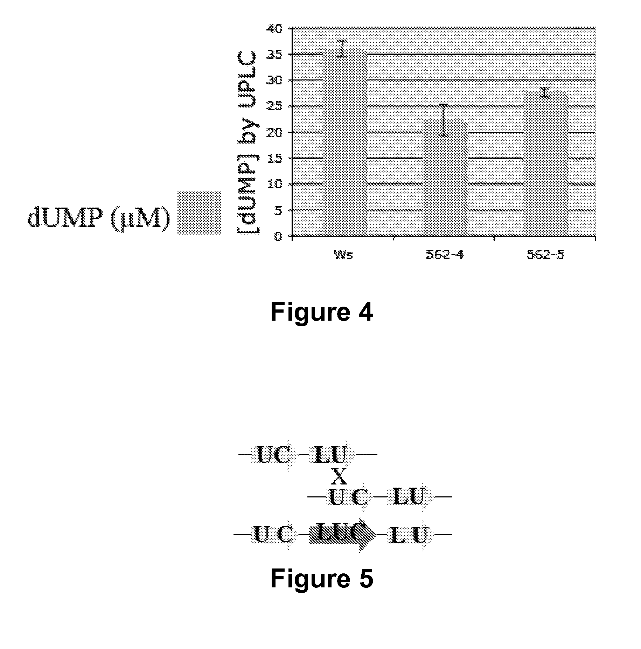

Figure 4 shows that the transformed plants have a lesser ability to transform dUTP to dUMP which definitely supports the idea that dUTPase activity was depleted in RNAi/*DUT1* plants, which are also sensitive to 5FU.

### Example 4: RNAi/DUT1 plants have better homologous recombination

Plants containing recombination substrate were provided by J. Molinier (Molinier et al., 2004). Line 58F used in this study carries a disrupted luciferase gene in a special arrangement designed specifically to reveal intermolecular recombination events (Figure 5). Line 58F is transformed with an empty control vector (RNAi/0) or by the RNAi*lDUT1* construct.
The detection of bioluminescence is performed on two-week-old seedlings from T2 generation of 4 independent lines for RNAi/*DUT1* plants (called -luc2, - luc4, -luc5 and -luc9).
Seedlings are sprayed with a solution of Luciferin (Luciferin 1 mM (Biosynth AG), triton X-100 0,05%) and visualized with a LCD camera used with the Photolite software. Number of spot per plant is then determined. Spots indicate presence of an active luciferase protein and hence occurrence of recombination events.

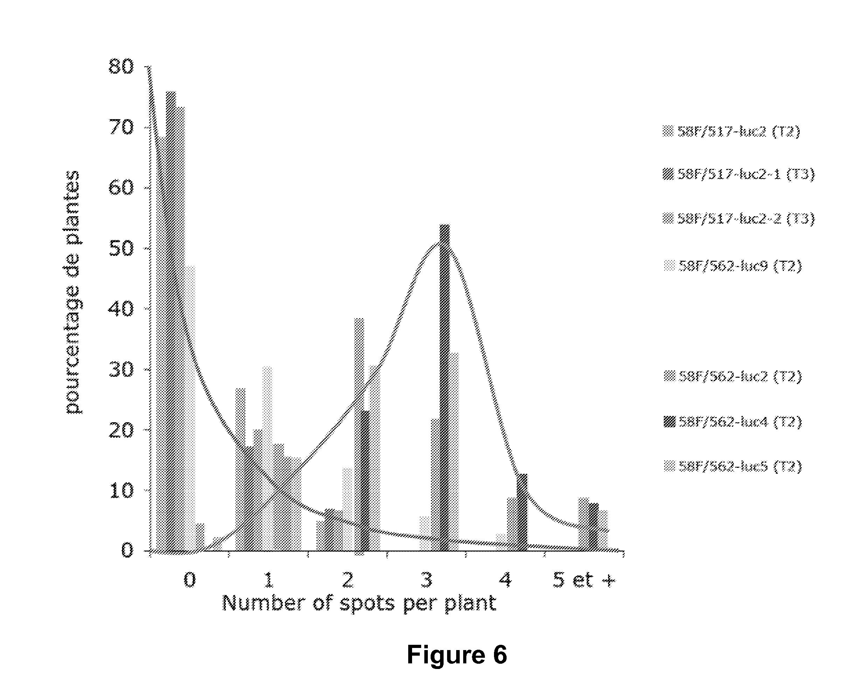

Figure 6 shows that the suppression of the At*DUT1* activity increases that occurrence of recombination events. It is to be noted that line 58F/562-luc9 grew well on 5-FU and is thus considered as not depleted in dUTPase activity, which parallels the fact that this line was not efficient in increasing somatic homologous recombination.

### Example 5: construction of RNAi constructs under an inducible promoter

The 307 bp pAlcA inducible promoter (Deveaux et al, 2003) was set into pPF111 at The SallHindlll sites. This left Sall and Spel sites downstream to the promoter, in which the RNAi/*DUT1* construct could be easily transfered following Xhol and Spel excision from pKannibal (Sall and Xhol have compatible cohesive ends). The T-DNA inducible RNAi/*DUT1* final construct was then introduced into a plant expressing the AlcR protein which becomes an activator for pAlcA under ethanol induction. The transformation is conducted prior to exposing the transformed plants to ethanol. The plants develop normally in the absence of ethanol. In the presence of ethanol, it is suggested to monitor induction, as a strong or prolonged induction may induce lethality of the transformed plants. Thus, it is preferred to induce the constructs for a short period of time, and only when suppression of DUT1 is sought.

### References

An et al. (1986), Plant Physiology, 81 :86-91
Aoyama T, Chua NH. (1997). Plant J.1 1 :605-12.
Babwah A. V., Waddell CS. (2000). Theor. Appl Genet. 100:802-809.
Bansal KC et al. (1992) Proc Natl Acad Sci U S A. Apr 15;89(8):3654-8.
Bechtold N, Ellis J, Pelletier G. (1993) C R Acad Sci 111. 316(10) : 1 194-1 199
Bevan MW et al. (1992). Biotechnology. 1992 24:367-70
Bleuyard JY, White Cl. (2004) EMBO J. Jan 28;23(2):439-49.
Christensen AH, Quail PH. (1996) Transgenic Res. May;5(3):213-8.
Chupeau, MC et al (1989). Biotechnology vol.7: pp. 503-508
Clough SJ and Bent AF (1998). Plant J 16: 735-743.
Depigny-This D, et al. (1992), Plant Mol Biol.;20(3):467-79.
Deveaux et al. (2003) Plant J. 36:918-30.
Doutriaux et al (1998) Mol Gen Genet. 257:283-91.
Dubouzet et al. (2003) Plant J. 2003 Feb;33(4):751 -63.
Eichholtz DA et al. (1987), Somat Cell Mol Genet. Jan;13(1 ):67-76
Erikson O., et al. (2004). Nat Biotechnol. Apr;22(4):455-8
Finer J. (1992) Plant Cell Report 11 : 323-328.
Gritz L, Davies J. (1983), Gene. Nov;25(2-3):179-88.
Guerche P, et al. (1987), Biochimie. Jun-Jul;69(6-7):621 -8.
Guillet M, Boiteux S. (2003) Mol Cell Biol. 23:8386-94.
Guillet et al. (2006) Nucleic Acids Res. 34:2056-66.
Hauptmann et al. (1988), Plant Physiology, 86 :602-606
Helliwell, C. and Waterhouse, P. (2003). Methods 30, 289-295
lida S, Terada R. (2005) Plant Mol Biol. 2005 Sep;59(1 ):205-19.
Ishida Y et al. (1996) Nature Biotechnol. 14, 745-50.
Kay et al. (1987), Science, 236 :1299-1302
Kizis D, Pages M. (2002) Plant J. Jun;30(6):679-89.
Klimyuk VI, Jones JD. (1997) Plant J. 11:1-14.
Koncz, C. and Schell, J. 1986. Mol. Gen.Genet. 204: 383-396.
Koo JC et al. (2004) Plant J. Feb;37(3):439-48.
Kouzminova EA, Kuzminov A. (2004) Mol Microbiol. 51:1279-95.
Lee SC et al. (2004) Mol Cells. Aug 31 ;18(1):107-14.
Matsuoka M et al. (1994) Plant J. Sep;6(3):31 1 -9.
McElroy,D. Zhang,W. Cao,J. Wu, R. (1990) Plant Cell 2(2):163
Meijer EG et al. (1991 ), Plant Mol Biol. 1991 May;16(5):807-20.
Miki B., McHugh S. (2004) Journal of Biotechnology 107 : 193-232
Molinier et al. (2004) Plant Cell. 16:342-52.
Moore I et al. (2006) Plant J. 45:651 -83.
Morita R et al. (2003) Plant Cell Physiol. 44:637-42.
Mukhopadhyay A, et al. (2004) Proc Natl Acad Sci USA.;101 (16):6309-14.
Mundy J, Chua NH. (1988) EMBO J. 1988 Aug;7(8):2279-86.
Norre F et al., Plant Mol Biol. 2002 Nov;50(4-5):699-712
Padidam M. (2003) Curr Opin Plant Biol. Apr;6(2):169-77.
Pellegrineschi A et al. (2004) Genome. Jun;47(3):493-500.
Pri-Hadash et al. (1992) Plant Cell. 4:149-59.
Rabbani MA et al. (2003) Plant Physiol. Dec;133(4):1755-67.
Roslan HA et al. (2001 ) Plant J. Oct;28(2):225-35.
Russell DA, Fromm ME. (1997) Transgenic Res. Mar;6(2):157-68.
Saijo Y et al. (2000) Plant J. Aug;23(3):319-27.
Saidi Y et al. (2005) Plant Mol Biol. 2005 Nov;59(5):697-71 1.
Sambrook J. et Russell D. W. (2001) "Molecular Cloning-A laboratory Manual, third edition", chapitre 1.1 , Editeur. : CSHL Press
Severin K, Schoffl F. (1990) Plant Mol Biol. Dec;15(6):827-33.
Shen Q, Uknes SJ, Ho TH. (1993) J Biol Chem. Nov 5;268(31 ):23652-60.
Shlomai J, Kornberg A. (1978) J Biol Chem. 253:3305-12
Siaud et al (2004) EMBO J. 231392-401.
Stevens R et al. (2004). Plant Cell. Jan;16(1 ):99-1 13. Epub 2003 Dec 5.
Straub PF, Shen Q, Ho TD. (1994) Plant Mol Biol. Oct;26(2):617-30.
Takahashi R, Joshee N, Kitagawa Y. (1994) Plant Mol Biol. Oct;26(1 ):339-52.
Terada R et al. (2004) Plant Cell Rep. Apr;22(9):653-9. Epub 2004 Jan 23.
Vilardell J et al. (1990) Plant Mol Biol. Mar;14(3):423-32.
Wang R, Zhou X, Wang X (2003) Transgenic Res. 2003 Oct;12(5):529-40.
White J et al. (1990) Nucleic Acids Res. 1990 Feb 25;18(4):1062.
Xu Y et al. (1995) Plant Mol Biol Mar;27(5):1060.
Yamaguchi-Shinozaki and Shinozaki. (1993) Mol Gen Genet. 236(2-3):331-40.
Yoshida K, et al. (1995) Appl Microbiol Biotechnol. Dec;44(3-4):466-72.
Young LW et al. (2005) Genome. Jun;48(3):547-55.
Zuo J, Chua NH. (2000) Curr Opin Biotechnol. Apr;11 (2):146-51.

## Claims

1. A plant presenting inhibition of the expression or activity of a dUTPase enzyme.

2. The plant of claim 1, wherein the sequence of said dUTPase comprises at least one sequence chose among SEQ ID N° 1 and SEQ ID N° 2.

3. The plant of claim 1 or 2, wherein said expression and/or activity of said dUTPase enzyme is inhibited by mutagenesis of the gene coding for said enzyme.

4. The plant of claim 1 or 2, which is a transgenic plant transformed with a DNA construct comprising a polynucleotide chosen among:
a) a polynucleotide coding for a dUTPase as specified in claim 2 ;
b) a polynucleotide which has a sequence complementary of the sequence of the polynucleotide of a) ;
c) a fragment of at least 12 consecutive nucleotides of a polynucleotide of a) or b), or capable of selective hybridization with a polynucleotide of a) or b) ;
d) a polynucleotide containing a polynucleotide X comprising at least 12 nucleotides, preferably at least 15, advantageously at least 20, and even more preferably at least 50 nucleotides capable of selective hybridization with the polynucleotide of a), and a polynucleotide Y that is complementary of said polynucleotide X,
under transcriptional control of an appropriate promoter.

5. The plant of any of claims 1 to 4, which is maize.

6. A method for improving recombination in a target plant as compared to a control plant, comprising totally or partially inhibiting expression and/or activity, in said target plant, of a dUTPase, wherein said expression and/or activity is not inhibited in said control plant.

7. The method of claim 6, wherein inhibition of said dUTPase is performed by using at least one polynucleotide chosen among :
a) a polynucleotide coding for a dUTPase as specified in claim 2 ;
b) a polynucleotide which has a sequence complementary of the sequence of the polynucleotide of a) ;
c) a fragment of at least 12 consecutive nucleotides of a polynucleotide of a) or b), or capable of selective hybridization with a polynucleotide of a) or b) ;
d) a polynucleotide usable for RNAi, containing a polynucleotide X comprising at least 12 nucleotides, preferably at least 15, advantageously at least 20, and even more preferably at least 50 nucleotides capable of selective hybridization with the polynucleotide of a), and a polynucleotide Y that is complementary of said polynucleotide X.

8. An expression cassette comprising a polynucleotide as specified in claim 7, under transcriptional control of an appropriate promoter.

9. A vector comprising the expression cassette of claim 8.

10. The vector of claim 9, further comprising a selectable marker gene under the control of an appropriate promoter, wherein said expression cassette and said selectable marker gene are flanked by homologous sequences Y5' and Y3'.

11. A method for integrating a DNA sequence X at a predetermined location within the genome of a plant, wherein said location is flanked by a sequence Y3' and a sequence Y5' respectively at its 3' and 5' ends comprising the steps of
a) providing to cells of said plant a nucleic acid comprising said DNA sequence X flanked by sequences Y3' and Y5' respectively at its 3' and 5' ends,
b) providing to said cells a polynucleotide as defined in claim 9 thereby leading to integration of said DNA sequence X within the plant genome by homologous recombination between said Y3' and Y5' sequences.

12. The method of claim 11, wherein said polynucleotide in step b) is provided via integration, within cells of said plant, of the expression cassette of claim 8.

13. The method of any of claim 11, wherein said polynucleotide in step b) is provided by crossing a transgenic plant containing said nucleic acid comprising said DNA sequence X flanked by sequences Y3' and Y5' respectively at its 3' and 5' ends, with another transgenic plant containing the sequence of said polynucleotide as defined in claim 7.

14. A method for excising a DNA fragment Z in the genome of a plant, wherein said DNA fragment Z is flanked by sequences Y3' and Y5' respectively at its 3' and 5' ends, wherein said sequences Y3' and Y5' are at least 80 % identical, comprising the steps of providing to said cells a polynucleotide as defined in claim 9, thereby leading to excision of said DNA fragment Z from the genome of said plant by recombination between said sequences Y3' and Y5'.

15. The method of any of claims 11 to 14, wherein said appropriate promoter is a meiosis specific promoter
